# EUROPEAN PATENT SPECIFICATION

(11) **EP 2 359 700 B1**
(45) Date of publication and mention of the grant of the patent: **07.05.2014**
(21) Application number: 11151354.5
(22) Date of filing: 19.01.2011
(51) Int. Cl.: A23L 1/29, A23L 1/308, A21D 6/00, A23L 1/00, A23L 1/0522, A23L 1/16, A23L 1/168, A23P 1/08, A61P 3/04, A61P 3/06, A61P 3/08, A61P 3/10, A23P 1/12

(54) **Method for manufacturing foodstuff comprising resistant starch for use in the treatment of metabolic syndrome and products therefrom**
Verfahren zur Herstellung von Lebensmitteln mit resistenter Stärke zur Anwendung bei Metabolischem Syndrom und entsprechende Lebensmittel
Procédé de fabrication d'aliments à base d'amidon résistant pour les appliquer à des symptômes métaboliques et produits ainsi fabriqués

(30) Priority: 21.01.2010 CN 201010003352
(43) Date of publication of application: 24.08.2011
(73) Proprietor: Camasone Co., Ltd., Taipei City, Chinese Taipei (CN)
(72) Inventor: Chuang, Lee-Ming, Taipei City (TW)
(74) Representative: Viering, Jentschura & Partner Patent- und Rechtsanwälte

(56) References cited:
- EP-A1- 0 749 697
- DE-A1-102004 056 337
- KIM J H ET AL: "Effect of extrusion conditions on resistant starch formation from pastry wheat flour", FOOD CHEMISTRY, ELSEVIER LTD, NL, vol. 99, no. 4, 1 January 2006 (2006-01-01), pages 718-723, XP025130046, ISSN: 0308-8146, DOI: DOI:10.1016/J.FOODCHEM.2005.08.054 [retrieved on 2006-01-01]
- CHOI BK ET AL: "Processed rice containing resistant starch for inhibiting adult diseases such as diabetes and obesity, and manufacturing method thereof", WPI/THOMSON,, vol. 2007, no. 40, 27 November 2006 (2006-11-27), XP002501574,

## Description

### Background

The present invention generally is related to a method for increasing the contents each of the non-digestible starches (for example, resistant starches and amylose) that are found in the cereal starch foodstuffs and a product made by the method, and especially related to a method for increasing the content of the non-digestible cereal foodstuffs for applying to the people acquired of metabolic syndromes.

### Prior Art

The prevalence of type II diabetes has been increased in the whole world years by years. This is caused by the metabolic disturbances usually found in the obese people whose quantities have increased recently and the people whose life styles have changed into those lacking physical activities and especially, caused by the disorder of the metabolisms of glucose and lipid. Those disorders are closely related to the occurrence of frequently found chronic diseases, for example, type II diabetes, hypertension, coronary heat disease (CHD) and so on. As an example, if an entity, affected with insulin resistance and low insulin-secretion, has a disorder of carbohydrate metabolism, then he or she is liable to acquire the diabetes. During the course of development of diabetes or in the early stage of diabetes, some other complications such as vascular diseases may occur so that the life quality of this entity will be threatened greatly. These disorders of metabolism will induce the increasing in terms of chronic diseases such as obesity, hyperglycemia, hypertension and dyslipidemia and they are collectively known as metabolic syndrome.

An individual affected with metabolic syndrome usually is associated with insulin resistance at the same time. The insulin resistance is considered as underlying cause of obesity, dyslipidemia, hypertension, glucose tolerance disorder, and metabolic syndromes. The metabolic pathway disorder caused by the insulin resistance will induce other syndromes caused by the metabolic disorders, for example, dyslipidemia, obesity, diabetes, hypertension, glucose tolerance disorder and other metabolic diseases. Other complications, such as stroke and cardiovascular disease, usually may coincide. In the past, fatness is used for predicating the indecent rate of metabolic syndromes because those who are obese are liable to having the insulin resistance. However, in fact, some people who have a body mass index (BMI) that does not reach the standard of fatness are affected with hyperinsulinemia, insulin resistance, type II diabetes, dementia chronic heart disease and so on. Thus, an individual who does not reach the fatness standard of the BMI still will be affected with metabolic syndrome.

As indicated by the literatures, one third or fourth of the adult people over 20 years old has the metabolic syndrome and the rate keeps on increasing. Recently, in Taiwan, about 30% to 65% of different age groups in people over 40 years old have such metabolic syndrome. All of the genetic abnormalities, fetal malnutrition and visceral adiposity may induce carbohydrate metabolism disorder and insulin resistance so as to cause the metabolic syndrome.

The glucose in the human body will keep invariable through conditioning the hormones. In case of hypoglycemia, the α -cells of pancreas will be promoted to secrete glucagon. On the other side, in case of hyperglycemia, the *β*-cells of pancreas will be promoted to secrete insulin. In case of hyperglycemia, after absorption of the exogenous glucose into liver, insulin will function to inhibit the glycogenolysis so that it is stored in form of glycogen. If the quantity of the so-stored glycogen reaches to saturation level (about 5% of liver weight), surplus of the exogenous glucose will continue forming fatty acids in liver by way of the function of insulin and then it will be transported, in form of lipoprotein, out of liver and into blood circulation. During the circulation, the fatty acids contained in the lipoprotein will be decomposed and the free fat acids will be released so that they will be utilized by other tissues including adipose and muscle tissues. And, these free acids will be used to form triglycerides. The decomposition of the triglycerides in the fat tissues will be inhibited through inhibiting the hydrolysis of triglycerides by the fat-splitting enzyme through the function of insulin. The aim of this function is for the absorption of glucose into the fat cells. At the same time, the glucose in these cells will be transformed into glycerol. The glycerol in the fat cells will combined with the free fatty acids transported from the liver forming triglycerides. Based on the above-mentioned mechanism, it is known that insulin, in addition to reducing the blood glucose, is closely related to the metabolism of lipids.

The metabolic syndrome may cause the metabolism disorders in terms of glucose and lipids so as to induce dyslipidemias, fatness, diabetes, insulin resistancet, and glucose intolerance, and to affect many other metabolic mechanisms. Thus, disorders are induced and some chronic diseases such as type II diabetes and cardiovascular diseases are further induced scarifying the health of the individual. In addition to using medicines, the prevention and controlling of the metabolic disorders can be effectively achieved by changing the diet habit or doing sports regularly, as indicated by many literatures.

In addition to dietary fibers that are proven effective in preventing and controlling the type II diabetes, the non-digestible starches have a health-care function similar to the dietary fibers. The so-called non-digestible fibers include resistant starch (RS) and amylose that is sorted as a type of starch owing to its composition. Comparing with the general starches, the amylose is without any branch in structure and thus the hydrolysis rate of amylose is slow. Further, owing to its physical structure and chemical characteristics, amylose is a kind of starch called as non-digestible starch because it can not be decomposed by enzymes (e.g. amylase) in the small intestine of the human body and only can be fermented by bacteria in the large intestine. Such a non-digestible starch will pass the stomach and the small intestine into the large intestine and then will be fermented into short-chained fatty acids by the intestinal flora. The non-digestible starches generally contain a high content of amylose. Owing to the characteristics of non-digestion and non-absorption, the physiological value and advantages of the non-digestible starches is the same as those of the dietary fibers.

Because the non-digestible starches has the similar characteristics to those of the dietary fibers, such as reducing the absorption rate of glucose, reducing the promotion rate of blood glucose after diet, reducing the concentration of total cholesterols and triglycerides in serum, promoting dyspepsia, promoting the output volume of the excrement, and promoting the contents of the short-chained fatty acids they can reduce the contents of the free fatty acids in the human body. Meanwhile, they also have some advantages in terms of, for example, promoting the oxidation of fat, reducing the pH in the intestines and low calorie. As shown by animal test, ingestion of the non-digestible starches in a long-term can improve the insulin sensitivity and can reduce the concentration of cholesterol and triglycerides in blood. Owing to the non-digestible characteristics, the non-digestible starches can be used as a bromatotherapy for preventing and treating the patients with metabolic syndrome.

The non-digestible starches can be made by a processing method. Thus, one object of the invention is to provide a method for processing and manufacturing cereal foodstuff in which the contents of the non-digestible starches is promoted after manufacturing and taste quality of the foodstuff is secured so as to apply to prevent and treat the metabolic syndrome.

DE 10 2004 056 337 describes a method for manufacturing pasta with hypoglycaemic properties for use in treating diabetes and cardiovascular disease, comprising pressure-kneading at a moisture content of between 20% to 60%, a temperature of between 100°C and 180°C and a pressure of between 1 and 8 bar, press-forming, and drying at a temperature of between 80°C and 90°C to a moisture content of about 12.5%.

Kim J.H. and Tanhehco E.J. (Food Chemistry 99 (2006) 718-723) describe a method for manufacturing wheat flour with an increased content of resistant starch for use in various functional foods, comprising preparing pastry wheat flour, press-kneading in a twin-screw extruder, wherein the ingredients are heated in different stages to 40°C, 60°C, 80°C, 100°C and 120° at a moisture content of 40%, and at an increased kneading pressure, press-forming, wherein the extrudates are cut, and drying at 50°C.

EP 0749 697 teaches the coating of grains to reduce glycaemic response.

### Summary

In one aspect, one method according to the present invention mainly includes a feed preparation step in which the ingredients are ground into powders and then screened by sieve sizes of 0.177 to 0.149 mm (80 to 100 meshes). Then, a press-kneading step is used to heat up and to press kneading the ingredients in sequence. The semi-finished products made in the second step is delivered to the output of the line, cooperating with a die and a blade, is conducted to form in a press-forming step. The formed pre-product is dried by an intermittent heating method in the drying step. Then, a binder is added to and sufficiently mixed with the semi-finished products to form a film coat on the surface of the pre-product and then is dried in a film-coat forming step.

In other aspect, another object of the present invention is to provide a method including a film-coat forming step in which some devices are used including a drying chamber. An input port connected with the drying chamber is used to deliver the kneaded-formed pre-products into the drying chamber. A blast hole is located under the drying chamber and upwardly blasts the hot air from the lower-side into the drying chamber to float the pre-products in the drying chamber 22 upwardly. More than one nozzle is located at a suitable place around the drying chamber so as to discharge the binder. The binder is sufficiently mixed with the pre-products in the drying chamber to form the film-coat. A series of outlet, located above the drying chamber, is used to blast the hot air to deliver the finished product out of the drying chamber.

In other aspect, another object of the present invention is to provide a method for producing the non-digestible starches that can be applied to the cereal foodstuff for curing metabolic syndromes. The composition of the product includes coarse rice, oat, corn, barley, buckwheat and wheat germ.

### Brief Description of the Drawings

Fig.1 is a flow chart showing the method for manufacturing the cereal foodstuff in accordance with the present invention.
Fig. 2 is a flow chart showing the method for forming the film-coat in accordance with the present invention.
Fig. 3A is a table showing the testing results regarding the change in glucose level in serum achieved at the first and the second day respectively acquired by the experimental group (numbered PPB-R-203) and the contrasting group (numbered TK9) in accordance with the human body clinical experiment of the product of the present invention.
Fig. 3B is a table showing the testing results regarding the change in glucose level in plasma, the achieved data at the third and the fourth day respectively acquired by the experimental group (PPB-R-203) and the control group (TK9) in accordance with the human body clinical experiment of the product of the present invention.
Fig. 4 is a curve diagram of Fig. 3A and Fig. 3B.
Fig. 5 shows the mean level of glucose in plasma each of the tested people of the experimental group (PPB-R-203) and the control group (TK9) at the first to the fourth day in accordance with the human body clinical experiment of the product of the present invention.
Fig. 6A shows the result data collected for four days in which the insulin level in serum was respectively taken from the people of experimental group (PPB-R-203) and the people of control group (TK9) in accordance with the human body clinical experiment of the product of the present invention.
Fig. 6B shows the result data collected for four days in which the insulin level in serum was respectively taken from the people of experimental group (PPB-R-203) and the people of control group (TK9) in accordance with the human body clinical experiment of the product of the present invention.
Fig. 7A is a curve diagram showing the change in the mean blood glucose level of the experimental group (PPB-R-203-2) and the control group acquired by regularly monitoring in accordance with the human body clinical experiment of the product of the present invention.
Fig. 7B is a bar graph showing the change in blood glucose level after meal for the tested people shown in Fig. 7A.
Fig. 8A is a curve diagram showing the change in mean blood glucose acquired by regularly monitoring the testing people in accordance with the human body clinical experiment of the product of the present invention.
Fig. 8B is a GI table achieved from the people of Fig. 8A having eaten the product PPB-R-203-3 of the present invention.

### Detailed Description of the Invention

This description is made in accordance with one preferred embodiment of the present invention.

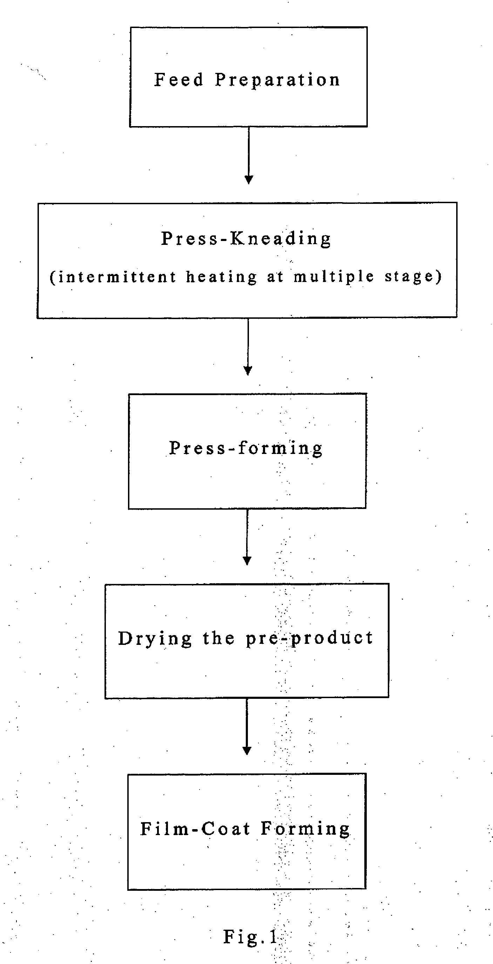

As shown in Fig. 1, coarse rice, oat, corn, barley, buckwheat and wheat germ are used as the ingredients and are processed by the steps described in the following paragraph.

A first step of feed preparation: all ingredients are ground into powder and then are screened by sieve sizes of 0.177 to 0.199 mm (80 to 100 meshes). A mixer is used to sufficiently mix the powder of ingredients evenly and then the ingredients are put into a barrel for reserving.

A second step of press-kneading: the ingredients made by the first step is intermittently heated and press-kneaded in a multiple stages together with setting a set of references including a moisture content of 40% in the ingredients, a temperature of from 30°C to 140°C, and a kneading pressure of 1mPa to 5mPa on a die. In this embodiment, this step is divided into seven stages and each stage has its own temperature reference as set forth in the following:
a first stage of from 85°C to 95°C;
a second stage of from 105°C to 115°C;
a third stage of from 110°C to 120°C;
a fourth stage of from 125°C to 135°C;
a fifth stage of from 130°C to 140°C;
a sixth stage of from 80°C to 90°C;
a seventh stage of from 35°C to 45°C.

The time period for heating in each one of the stage is set to be from 1 min to 5 min.

A third step of press-forming: the pre-product made by the second step is pre-formed into a desired product form and then is cut into the desired form by a die located at an outlet of a pressing line, cooperating with a blade. In this embodiment, the rotating speed of the blade is from 20Hz to 35Hz for conducting cutting-forming function. The product so-made is in form of grain. However, if the blade is operated with a speed of 5Hz to 20Hz to conduct the cutting function, a form of bar will be achieved.

A fourth step of drying: the formed pre-product is intermittently heated under setting a temperature reference of from 30°C to 70°C. The pre-product, after drying, has a moisture content of form 5% to 15%.

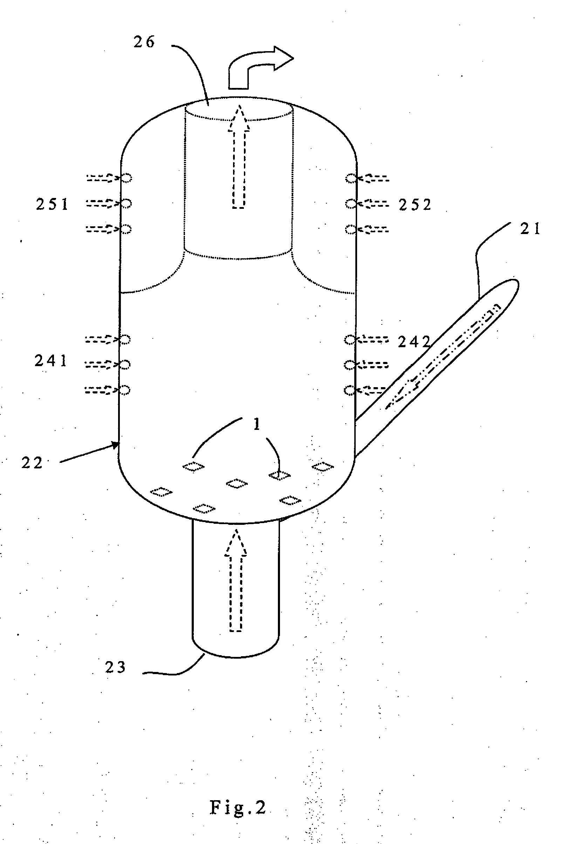

A fifth step of film-coat-forming: as shown in the flow chart of Fig.2, the pre-product 1 produced in the fourth step is delivered into drying chamber 22 through input port 21. Drying chamber 22 is equipped with a blast hole 23 on lower side. The hot air is upwardly blown from the lower side into chamber 22. Thus, the pre-product 1 may float upwardly in chamber 22. In addition, the binder is discharged through nozzles 241, 242, 251 and 252 located around chamber 22. Thus, the binder can be sufficiently mixed with the pre-product 1 and form a film-coat on the surface of pre-product 1. The hot air through the blast hole 23 will upwardly blow the pre-product having been mixed with the binder into a higher side of drying chamber 22 and then the pre-product is delivered out of chamber 22 through outlet port 26 on the higher side of drying chamber 22. The product made in accordance with this step will have a moisture content of 15% to 20%.

The cereal foodstuff made in accordance with the five steps of this embodiment is numbered as PPB-R-203. It has a form of ordinary rice and can be cooked by the way for cooking rice. If the die and the rotating speed in the fourth step are changed, the predicated form of the product in appearance will be a different form such as a bar form which can be cooked by the way for cooking noodle.

The product PPB-R-203 made in accordance with the method of the embodiment of the invention mentioned above is sent to conduct tests by Medicine and Industrial Hygiene Certification Laboratory of HWAYO TECH & LAB CO., LTD. A test report showing the calorie and nutrition ingredient in 100 grams of the product is as shown in Table 1.

**Table 1**

| Tested Item | Testing Results (per 100 grams) |
|---|---|
| calorie | 350 Cal |
| protein | 14.7 g |
| fat | 1.27 g |
| Saturated fat | 0.21g |
| Anti-form fat | 0 g |
| carbohydrate | 69.9 g |
| sodium | 107 mg |

As shown in Table 1, the product made according to this embodiment is similar to the ordinary rice in terms of calorie and nutrition ingredients per 100 grams. That is, the product made in this embodiment can replace the rice to serve as a human calorie source.

In addition, the ingredients of PPB-R-203 product is tested to measure the amylose (that is, non-digestible starches) contained therein. The results are shown in Table 2.

**Table 2**

| Tested Item | Tested Results |
|---|---|
| Amylose | Non-detectable |

PPB-R-203 product is tested to measure the amylose (that is, non-digestible starches) contained therein. The results are shown in Table 2.

**Table 3**

| Tested Item | Tested Results |
|---|---|
| Amylose | 27.45% |

As shown in Table 3, the product of PPB-R-203 made in accordance with this embodiment has an amylose content as high as 27.45%. Comparing with the results shown in Table 3 (the tested results for the ingredients used in this embodiment), it is proved that the cereals without amylose originally can be made into the foodstuff having high-content of amylose by the method of the present invention. Thus, the content of non-digestible starches in the foodstuff is promoted indeed.

As to applying the product made in accordance with the method of the present invention to metabolic syndromes, PPB-R-203 product made by the method of the present invention is set as the experimental group while the Taiwanese stem rice (no. Tk9) as the control group. 40 health people are recruited to serve as the researching objects with regularly testing the change of blood glucose and the change of insulin in blood for a term of 4 days conducting a clinical experiment "A Study to Evaluate the Efficacy and Safety of PPB-R-203-Based Meal Versus TK9-Based Meal in 20 Patients With Diabetes "numbered NCT01065402(IRC01080520).

In this test, the 40 healthy people are randomly separated into two diet modules in which one is the experimental group (no. PPB-R-203) and the other is the control group (no. TK9). Each module continues for two days and totally is tested for four days. A standard menu is used for the tested people to ensure that the quantities each of calorie and the three nutrients achieved from diets are the same. The time for serving the diets are respectively at 7:00 (breakfast), 12:00 (lunch), 17:00 (dinner) and 22:00 (midnight snack).

In this case, the glucose level in plasma (mg/dL) and the insulin level in serum (*µ* U/mL) are used as the testing index for regulating the blood glucose. The samples are respectively collected at the time 30 min before breakfast and at the time 15, 30, 45, 60, 90, 120 and 180 min after diet. As to lunch and dinner, the samples are respectively collected at the time 30 min before diet and 2 hr after diet so as to observe the varied level of the testing index per day.

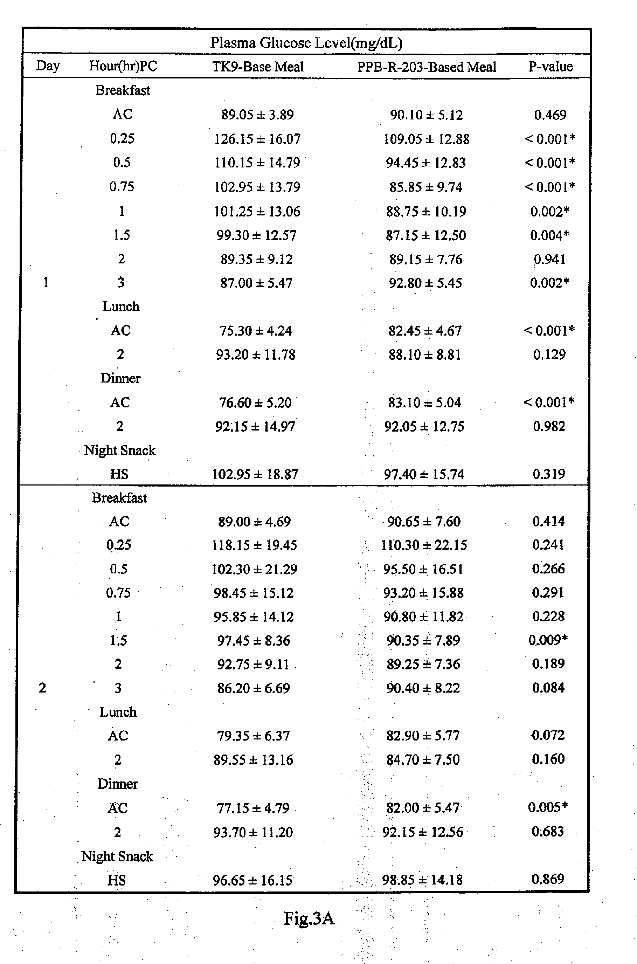

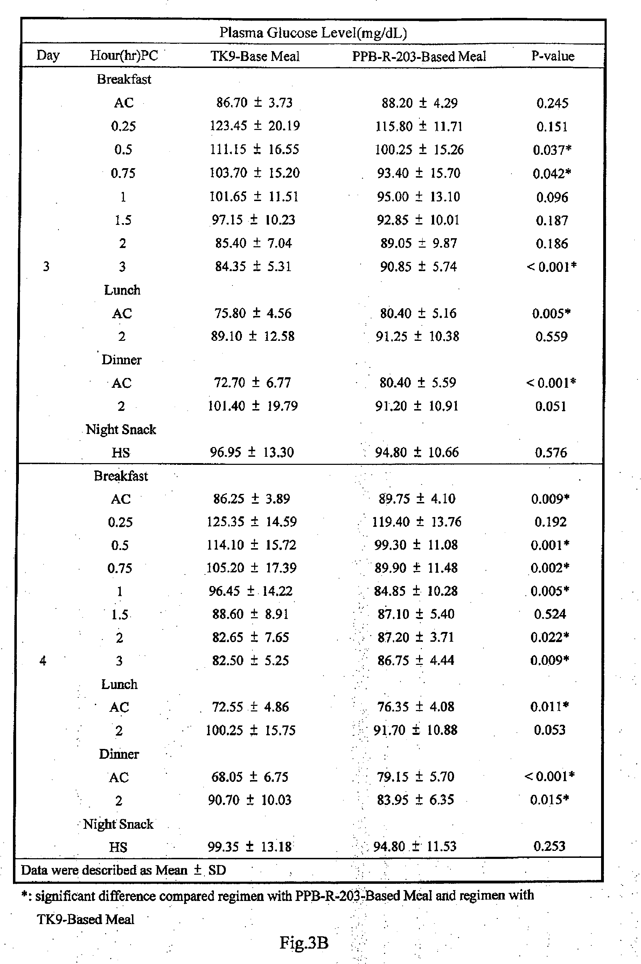

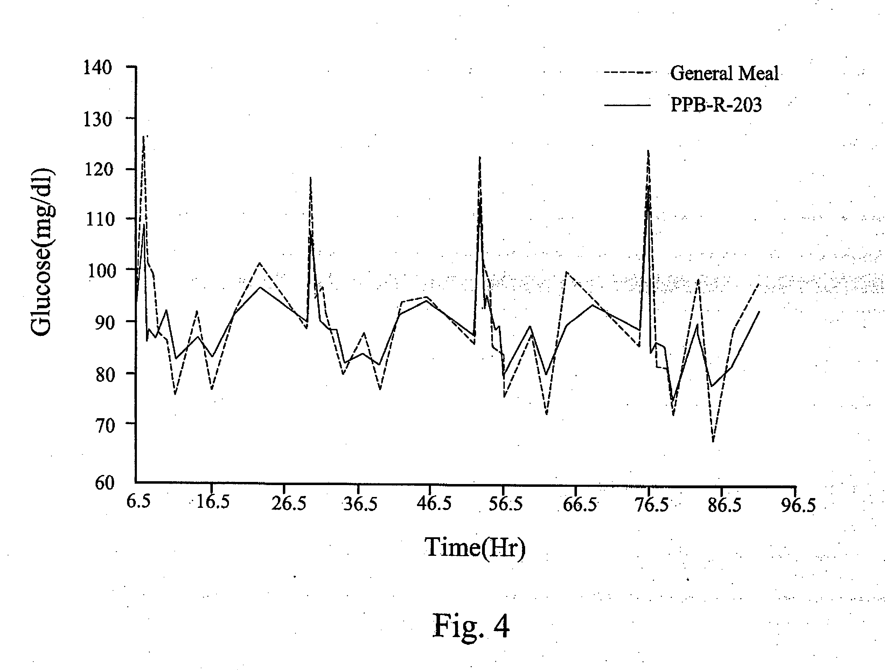

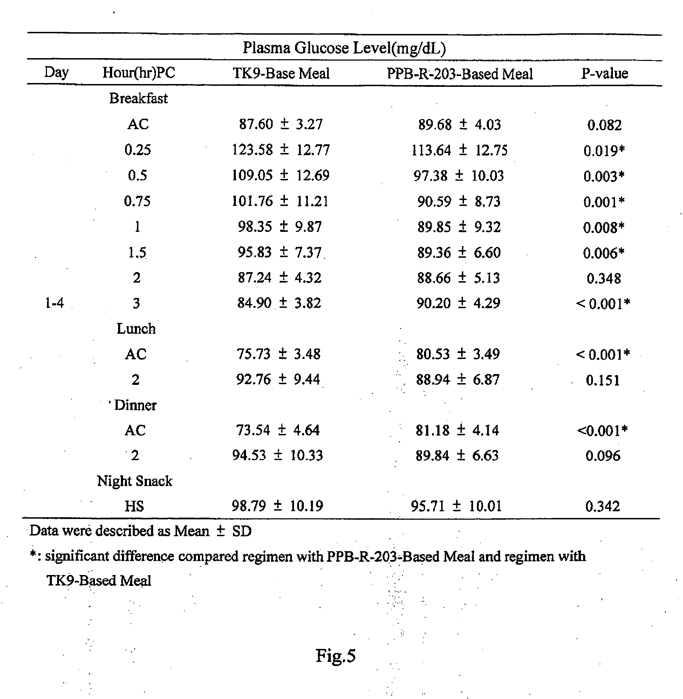

As to the results regarding the change in glucose level in serum, the achieved data at the first and the second day is shown in Fig. 3A. The data for the third and the fourth day is shown in Fig. 3B. Fig 4 shows the curve diagram indicating the change of blood glucose for the people having testing data as shown in Fig. 3A and Fig. 3B. In this case, Y axis means the level of blood glucose and the X axis means the time when the test is conducted. Fig. 5 shows the mean level of glucose in serum of the tested people tested at the first to the fourth day.

As shown in Fig. 5, regarding the mean level of glucose in serum of the tested people at the first to the fourth day, the mean level found in the people of the experimental group (who have eaten PPB-R-203 product made in accordance with the method of the present invention) is obviously lower than that found in the people of the control group (no. TK9) tested at the time 0.25 hr to 1.5 hr after breakfast (*p* < 0.05). 0.25 hr after diet, the glucose level in serum is lowered from 123.58 mg/dL to 113.64 mg/dL. The glucose levels in serum tested at the time 0.5 hr before diet are respectively promoted from 75.73 mg/dL to 80.53 mg/dL (lunch) and from 73.54 mg/dL to 81.18 mg/dL (dinner).

As indicated by Fig. 3A, Fig. 3B, Fig.4 and Fig.5, it can be known that the tested people having eaten the product made in according to the method of the present invention (PPB-R-203) will have a lower glucose level in serum after diet, thus, indicating that their glucose tolerance is better. At the period between each meal time, the one who has eaten the product of the invention (PPB-R-203) has a higher blood glucose level before meal, thus, indicating that the product of the invention can mitigate the absorption of the blood glucose in human body and maintain a constant blood glucose when the one is on an empty stomach. Based on the above mentioned, it is proved that the physiological mechanism of the one who eats the product of the present invention is affected during the human experiment, for example, mitigating the absorption rate of glucose after meal and lowering down the promotion rate of blood glucose after meal.

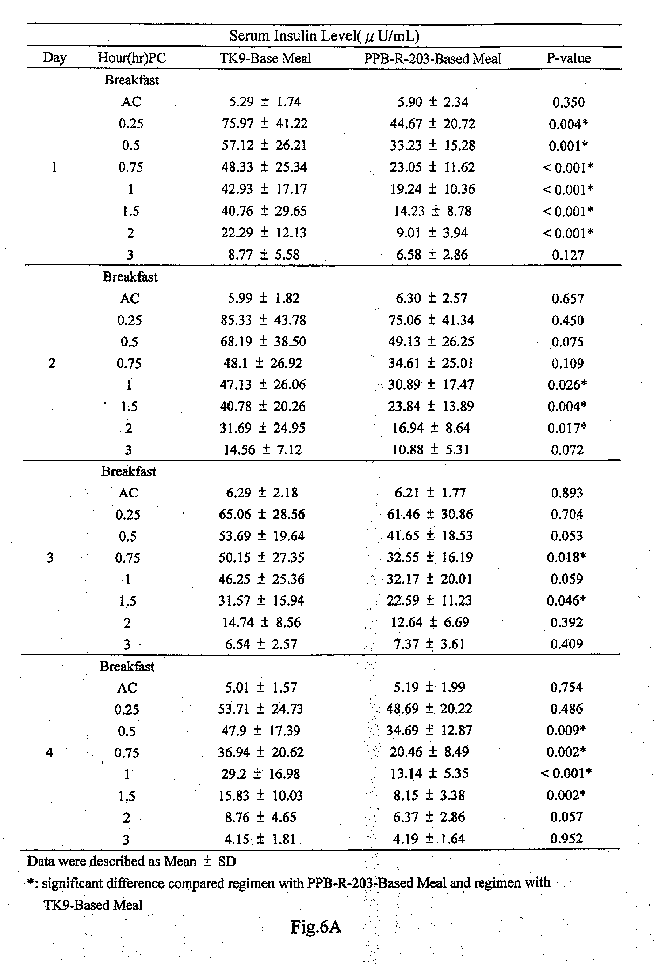

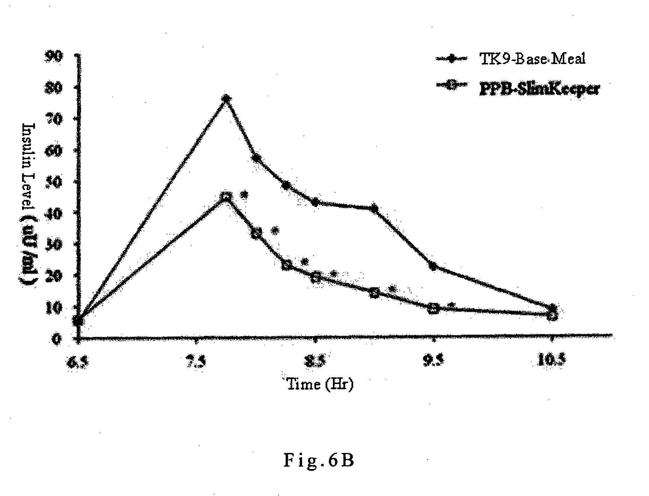

As shown in Fig. 6A, the result data collected for four days were the insulin level in serum respectively taken from the people of experimental group (PPB-R-203) and the people of control group (TK9). Fig. 6B shows the curve diagram indicating the change of insulin level of the tested people wherein Y axis means the insulin level and X axis means the time at which the test was conduct. Based on the statistics, the people of the experimental group (who have eaten PPB-R-203 product made in accordance with the method of the present invention) have an insulin level in serum obviously lower than that of the people of the control group (TK9) tested at the time 0.25 hr to 2 hr after breakfast at the first testing day (p < 0.05). The insulin level at the time 0.25 hr after meal is lowered from 75.97 *µ*U/mL to 44.67 *µ*U/mL. In other testing items, the insulin level in serum of the people of the experimental group (PPB-R-203) is trended to be lower than that of the people of the control group (TK9).

When the blood glucose level in human body is lowered or the insulin sensitivity to blood glucose is promoted, the quantity of insulin required by the human body decreases. Thus, the secretion amount of insulin will reduce so as to lower down the insulin level in blood. Therefore, based on the results shown in Fig. 6A and Fig. 6B, the one who has eaten the product of the present invention (PPB-R-203) will have a lower insulin level in his body and this indicates that the insulin secretion of the tested people is lower. Based on the above mentioned, the product made in accordance with the present invention will affect the physiological mechanism as proved by the human experiment and indeed, it can increase the insulin sensitivity to blood glucose together with lowering down the insulin secretion in body.

A product numbered PPB-R-203-2 made in accordance with another embodiment of the present invention has a form in bar. The people who eat the PPB-R-203-2 product respectively at three meal time are recruited in the experimental group and other people eating the noodle available in market are recruited in the control group. There are 24 diabetes patients serving as the testing objects and the change in blood glucose of each tested people is regularly measured for two days so as to conduct a clinical test "A Study to Evaluate the Blood Glucose Regulation and Safety of PPB-R-203-02 Based Noodle Versus Commercially Available Wet Noodle in Patients With Diabetes" numbered NCT01102452(IRC01100118).

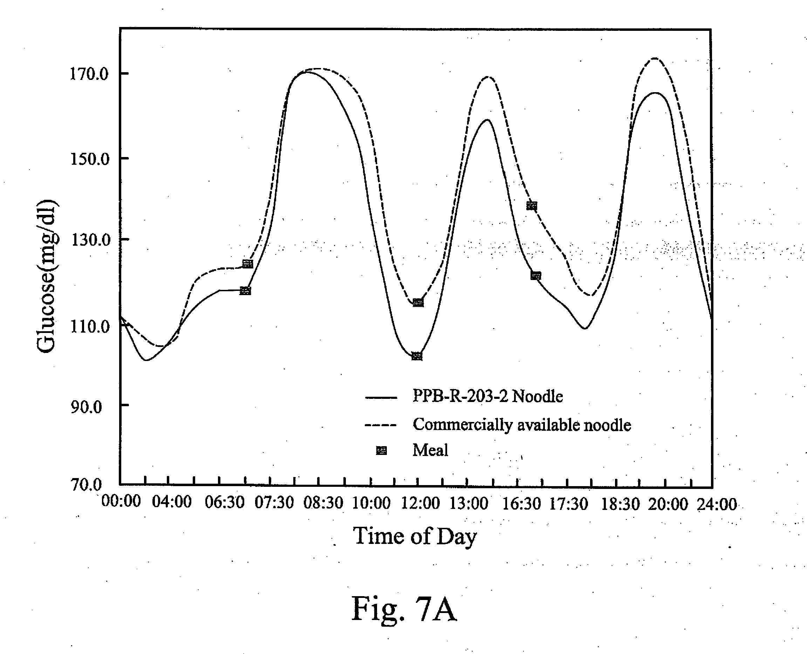

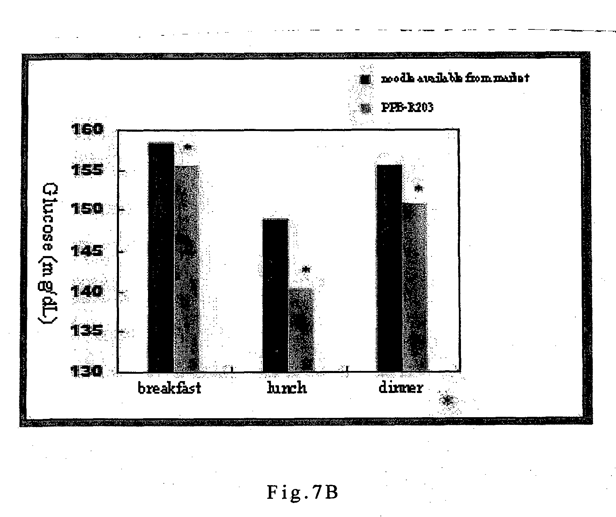

The tested results are shown in Fig. 7A which is a curve diagram showing the change in the mean blood glucose level of the tested people (including the experimental group and the control group) regularly monitored by the Medtronic MiniMed CGMS device continuing for two days. Fig. 7B is a bar graph showing the change in blood glucose level after meal for the tested people shown in Fig. 7A. As indicated by the tested results, comparing with the control group, the people of the experimental group having eaten the product of PPB-R-203-2 achieve long-term effects in terms of controlling the blood glucose level rendering that a diabetes patient will sustain a lower blood glucose level after meal and will have a lower span of change in blood glucose.

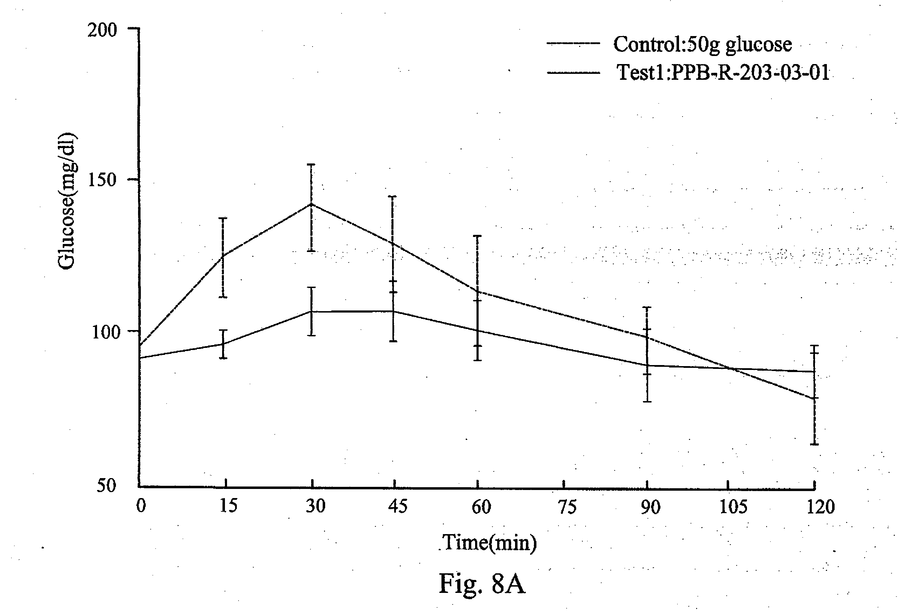

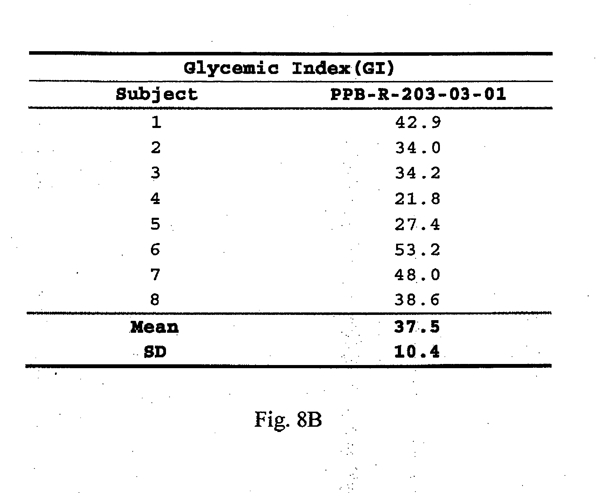

A product numbered PPB-R-203-3 made in accordance with another embodiment of the present invention has a platy form similar to a cookie. 8 healthy adults serve as the testing objects conducting a clinical experiment "The Report of A Comparative Study to Evaluate the Glycemic Response of Five Kinds of PPB-R-203-03 Crackers in Healthy Non-diabetic Adults" numbered IRC01090928. The oral glucose tolerance test is used to be the testing method. That is, the blood glucose reaction of the tested people was tested at the time 2 hr after he eats the product PPB-R-203-3 and at the time 2 hr after he eats 50 g glucose so as to measure the glycemic index (GI) of the tested people having the results as shown in the GI curve diagram of Fig. 8A. The lower the GI is, the lower the reaction of blood glucose is. Fig. 8B is a GI table achieved from the people who have eaten the product PPB-R-203-3 of the present invention having a mean value of 37.5. Thus, the blood glucose reaction caused by eating the product of the present invention is so low that it can achieve an effect in controlling the metabolic syndromes. Based on the results acquired from the product analysis and the above-mentioned human clinical experiment, cereals originally containing not any non-digestible starch such as amylose can be made into the foodstuff having high contents of amylose in accordance with the product made by the method of the present invention. Thus, the content of non-digestible starches in the foodstuff is promoted indeed. Furthermore, based on the results acquired in the embodiment of the present invention, the clinical experiments each numbered NCT01065402 (IRC01080520), NCT01102452(IRC01100118) and IRC01090928, the product made in accordance with the present invention will confirmedly mitigate the absorption of carbohydrates by human body, the absorption rate of glucose after meal and the promotion rate of blood glucose after meal together with improving the consistency of blood glucose at an empty stomach. Furthermore, it is proved by the test of healthy people that the product will affect the physiological mechanisms, can promote the insulin sensitivity and can reduce the secretion amount of insulin. Thus, the product made in accordance with the present invention can be applied to the dietary therapy to prevent and cure the diabetes in the future so as to control the blood glucose more efficiently endowing benefits to the patients of metabolic syndrome.

## Claims

1. A method for manufacturing a special starch foodstuff for use in the treatment of metabolic syndrome, comprising:
a first step of feed preparation, wherein all cereal ingredients of starch type are ground into powders screened by sieve openings of 0.177 to 0.149 mm (80 to 100 meshes) and are sufficiently mixed for reserving;
a second step of press-kneading, wherein the ingredients made by the first step are heated and press-kneaded orderly with setting a set of references including a moisture content of 30% to 50% in the ingredients, a temperature of from 30°C to 140°C, and a kneading pressure of 1 MPa to 5 MPa on a die, wherein the press-kneading step comprises:
a first stage having a temperature reference set from 85°C to 95°C;
a second stage having a temperature reference set from 105°C to 115°C;
a third stage having a temperature reference set from 110°C to 120°C;
a fourth stage having a temperature reference set from 125°C to 135°C;
a fifth stage having a temperature reference set from 130°C to 140°C;
a sixth stage having a temperature reference set from 80°C to 90°C;
a seventh stage having a temperature reference set from 35°C to 45°C; and
wherein a heating time in each one of the stage is set to be from 1 min to 5 min;
a third step of press-forming, wherein a pre-product made by the second step is pre-formed into a desired product form and then is cut into the desired form by a die located at an outlet of a pressing line, cooperating with a blade;
a fourth step of drying, wherein the formed pre-product is intermittently heated under setting a temperature reference of from 30°C to 70°C to produce a pre-product having a moisture content of from 5% to 15%;
a fifth step of film-coat-forming, wherein a binder is added to and sufficiently mixed with the pre-product produced in the fourth step to form a film coat on a surface of the pre-product, and then is dried to produce a product; and
wherein the product so-made has a moisture content of from 15% to 20%.

2. The method for manufacturing a special starch foodstuff for use in the treatment of metabolic syndrome claimed in claim 1, wherein devices used in the step of film-coat-forming comprises:
a drying chamber;
an input port connected with the drying chamber to deliver the pre-product into the drying chamber;
a blast hole located under the drying chamber to upwardly blast a hot air from a lower side into the drying chamber;
more than one nozzle located at a suitable place around the drying chamber to discharge a binder to sufficiently mix with the pre-product in the drying chamber; and
an outlet port located above the drying chamber to deliver the product out of the drying chamber.

3. The method for manufacturing a special starch foodstuff for use in the treatment of metabolic syndrome claimed in claim 2, wherein the film-coat-forming step comprises:
a first step wherein the pre-product made in the fourth step of drying is delivered into the drying chamber and the hot air is upwardly blown into the drying chamber from the lower side so that the pre-product can upwardly float in the drying chamber;
a second step of discharging the binder to sufficiently mix with the pre-product so as to form the film-coat; and
a third step wherein the product having the film-coat formed thereon is delivered out of the drying chamber by the hot air.

4. A foodstuff product for use in the treatment of a metabolic syndrome obtainable by the method of claim 1 having a composition comprising:
a coarse rice of 20% to 60% by weight;
an oat of 5% to 30% by weight;
a corn of 10% to 40% by weight;
a barley of 10% to 30% by weight;
a wheat of 10% to 30% by weight; and
a buckwheat of 5% to 15% by weight.

## Patentansprüche

1. Ein Verfahren zur Herstellung eines speziellen Stärke-Lebensmittels zur Verwendung in der Behandlung von metabolischem Syndrom, umfassend:
einen ersten Schritt der Futterzubereitung, wobei alle Getreidebestandteile vom Stärketyp zu Pulver zermahlen, durch Sieböffnungen von 0,177 bis 0,149 mm (80 bis 100 Mesh) gesiebt und zur Aufbewahrung ausreichend vermischt werden;
einen zweiten Schritt des Pressknetens, wobei die Inhaltsstoffe, die durch den ersten Schritt hergestellt wurden, erhitzt und planmäßig durch das Einstellen einer Reihe von Referenzwerten einschließlich einem Feuchtigkeitsgehalt der Inhaltsstoffe von 30 % bis 50 %, einer Temperatur von 30° C bis 140° C und einem Knetdruck von 1 MPa bis 5 MPa durch eine Düse pressgeknetet werden, wobei der Pressknetschritt umfasst:
eine erste Stufe, die eine Temperatureinstellung von 85° C bis 95° C hat;
eine zweite Stufe, die eine Temperatureinstellung von 105° C bis 115° C hat;
eine dritte Stufe, die eine Temperatureinstellung von 110° C bis 12° C hat;
eine vierte Stufe, die eine Temperatureinstellung von 125° C bis 135° C hat;
eine fünfte Stufe, die eine Temperatureinstellung von 130° C bis 140° C hat;
eine sechste Stufe, die eine Temperatureinstellung von 80° C bis 90° C hat;
eine siebte Stufe die eine Temperatureinstellungen von 35° C bis 45° C hat; und
wobei die Heizzeit in jeder der Stufen auf 1 min bis 5 min eingestellt wird;
einen dritten Schritt des Pressformens, wobei ein Vorprodukt, das durch den zweiten Schritt hergestellt wurde, in eine gewünschte Produktform vorgeformt wird und dann durch eine Düse, die sich am Ausgang einer Presslinie befindet, im Zusammenwirken mit einer Klinge in die gewünschte Form geschnitten wird;
einen vierten Schritt des Trocknens, in dem das gebildete Vorprodukt periodisch unter Temperatureinstellungen von 30° C bis 70° C erhitzt wird, um ein Vorprodukt mit einem Feuchtigkeitsgehalt von 5 % bis 15 % zu erzeugen;
einen fünften Schritt der Filmschichtbildung, wobei dem Vorprodukt, das durch den vierten Schritt erzeugt wurde, ein Bindemittel beigefügt und mit diesem genügend vermischt wird, um eine Filmbeschichtung auf einer Oberfläche des Vorproduktes zu erzeugen, das dann getrocknet wird, um ein Produkt zu erzeugen; und
wobei das auf diese Weise hergestellte Produkt einen Feuchtigkeitsgehalt von 15 % bis 20% hat.

2. Das Verfahren zur Herstellung eines speziellen Stärke-Lebensmittels zur Verwendung in der Behandlung von metabolischem Syndrom nach Anspruch 1, wobei das in dem Schritt der Filmschichtbildung verwendete Gerät umfasst:
eine Trocknungskammer;
eine Eingangsöffnung verbunden mit der Trocknungskammer, um das Vorprodukt in die Trocknungskammer zu führen;
ein Gebläseloch, das sich unter der Trocknungskammer befindet, um Heißluft von der unteren Seite aufwärts in die Trocknungskammer zu blasen;
mehr als eine Düse, die sich an einer geeigneten Stelle rund um die Trocknungskammer befindet, um ein Bindemittel abzugeben und dieses mit dem Vorprodukt in der Trocknungskammer in ausreichendem Maße zu mischen, und
eine Ausgangsöffnung, die sich oberhalb der Trocknungskammer befindet, um das Produkt aus der Trocknungskammer zu entlassen.

3. Das Verfahren zur Herstellung eines speziellen Stärke-Lebensmittels zur Verwendung in der Behandlung von metabolischem Syndrom nach Anspruch 2, wobei die Filmschichtbildung umfasst:
einen ersten Schritt, wobei das Vorprodukt, das im vierten Schritt durch Trocknung hergestellt wurde, in die Trocknungskammer geführt wird und die Heißluft von der unteren Seite aufwärts in die Trocknungskammer geblasen wird, so dass das Vorprodukt in die Trocknungskammer aufwärts schweben kann;
einen zweiten Schritt des Abgebens des Bindemittels, um dieses ausreichend mit dem Vorprodukt zu mischen und so die Filmschicht zu bilden, und
einen dritten Schritt, wobei das Produkt, das die auf ihr gebildete Filmschicht aufweist, durch die Heißluft aus der Trocknungskammer entlassen wird.

4. Ein Lebensmittelprodukt zur Verwendung in der Behandlung von metabolischem Syndrom, wobei das Lebensmittelprodukt durch das Verfahren nach Anspruch 1 erhalten wird und eine folgende Zusammensetzung aufweist, die umfasst:
einen groben Reis mit 20 bis 60 Gewichtsprozent;
einen Hafer mit 5 bis 30 Gewichtsprozent;
einen Mais mit 10 bis 40 Gewichtsprozent;
eine Gerste mit 10 bis 30 Gewichtsprozent;
einen Weizen mit 10 bis 30 Gewichtsprozent; und
einen Buchweizen mit 5 bis 15 Gewichtsprozent.

## Revendications

1. Procédé de fabrication d'aliments spéciaux à base d'amidon destinés à une utilisation dans le traitement du syndrome métabolique, comprenant :
une première étape de préparation d'un aliment, dans laquelle tous les ingrédients à base de céréales du type amidon sont broyés en poudres tamisées à travers des ouvertures de tamis de 0,177 à 0,149 mm (80 à 100 mesh) et sont suffisamment mélangés pour être réservés ;
une deuxième étape de pétrissage sous presse, dans laquelle les ingrédients fabriqués dans la première étape sont chauffés et pétris sous presse de façon méthodique avec la fixation d'un jeu de références comprenant un taux d'humidité de 30 % à 50 % dans les ingrédients, une température de 30 °C à 140 °C et une pression de pétrissage de 1 MPa à 5 MPa sur une matrice, dans laquelle l'étape de pétrissage sous presse comprend :
un premier stade ayant une température de référence fixée de 85 °C à 95 °C ;
un deuxième stade ayant une température de référence fixée de 105 °C à 115°C;
un troisième stade ayant une température de référence fixée de 110 °C à 120°C;
un quatrième stade ayant une température de référence fixée de 125 °C à 135°C;
un cinquième stade ayant une température de référence fixée de 130 °C à 140 °C ;
un sixième stade ayant une température de référence fixée de 80 °C à 90°C;
un septième stade ayant une température de référence fixée de 35 °C à 45 °C ; et
dans lequel le temps de chauffage de chacun des stades est fixé de 1 min à 5 min ;
une troisième étape de formation sous presse, dans laquelle un pré-produit fabriqué dans la deuxième étape est préformé sous forme d'un produit souhaité et est ensuite découpé à la forme souhaitée par une matrice située à une sortie de la ligne de compression, en coopération avec une lame ;
une quatrième étape de séchage, dans laquelle le pré-produit formé est chauffé par intermittence par fixation d'une température de référence de 30 °C à 70 °C pour produire un pré-produit ayant un taux d'humidité de 5 % à 15 % ;
une cinquième étape de formation d'une couche en film, dans laquelle un liant est ajouté au pré-produit et suffisamment mélangé avec ledit pré-produit produit dans la quatrième étape pour former une couche en film sur une surface du pré-produit et est ensuite séché pour produire un produit ; et
dans lequel le produit ainsi fabriqué a un taux d'humidité de 15 % à 20 %.

2. Procédé de fabrication d'aliments spéciaux à base d'amidon destinés à une utilisation dans le traitement du syndrome métabolique selon la revendication 1, dans lequel les dispositifs utilisés dans l'étape de formation d'une couche en film comprennent :
une chambre de séchage ;
une ouverture d'entrée reliée à la chambre de séchage pour délivrer le pré-produit dans la chambre de séchage ;
un trou de coulée situé sous la chambre de séchage pour souffler de bas en haut de l'air chaud depuis un côté inférieur dans la chambre de séchage ;
plus d'une buse située en un endroit convenable autour de la chambre de séchage pour décharger un liant pour qu'il soit suffisamment mélangé avec le pré-produit dans la chambre de séchage ; et
une ouverture de sortie située au-dessus de la chambre de sortie pour délivrer le produit hors de la chambre de séchage.

3. Procédé de fabrication d'aliments spéciaux à base d'amidon destinés à une utilisation dans le traitement du syndrome métabolique selon la revendication 2, dans lequel l'étape de formation d'une couche en film comprend :
une première étape dans laquelle le pré-produit fabriqué dans la quatrième étape de séchage est délivré dans la chambre de séchage et de l'air chaud est soufflé de bas en haut dans la chambre de séchage depuis le côté inférieur de sorte que le pré-produit puisse flotter de bas en haut dans la chambre de séchage ;
une deuxième étape de décharge du liant pour qu'il soit suffisamment mélangé avec le pré-produit de façon à former une couche en film ; et
une troisième étape dans laquelle le produit sur lequel est formée une couche en film est délivré hors de la chambre de séchage par l'air chaud.

4. Produit alimentaire destiné à une utilisation dans le traitement d'un syndrome métabolique susceptible d'être obtenu par le procédé de la revendication 1, ayant une composition comprenant :
du riz grossier à raison de 20 % à 60 % en poids ;
de l'avoine à raison de 5 % à 30 % en poids ;
du maïs à raison de 10 % à 40 % en poids ;
de l'orge à raison de 10 % à 30 % en poids ;
du blé à raison de 10 % à 30 % en poids ; et
du blé noir à raison de 5 % à 15 % en poids.
